# EUROPEAN PATENT SPECIFICATION

(11) **EP 3 145 433 B1**
(45) Date of publication and mention of the grant of the patent: **04.07.2018**
(21) Application number: 15753490.0
(22) Date of filing: 10.05.2015
(51) Int. Cl.: A61B 90/00, F21W 131/20, F21V 21/40, F21W 131/205

(54) **UNIVERSAL HANDLE COVER**
UNIVERSELLE GRIFFABDECKUNG
COUVERCLE DE LA POIGNÉE

(30) Priority: 21.05.2014 US 201462001089 P
(43) Date of publication of application: 29.03.2017
(73) Proprietor: D.M. Benatav Ltd., Petah Tikva, 4927934 (IL)
(72) Inventor: Benatav, Dror, 6941402 Tel Aviv (IL)
(74) Representative: Suèr, Steven Johannes
(86) International application number: PCT/IL2015/050482
(87) International publication number: WO 2015/177782

(56) References cited:
- US-A- 4 976 299
- US-A1- 2014 071 701
- US-B2- 7 757 352

## Description

### TECHNICAL FIELD

The present invention relates generally to the disposable handle covers, particularly sterile disposable handle covers with radiating fingers connected with breakable ribs.

### BACKGROUND

Sterility of medical equipment, particularly operating room equipment, is of great importance in preventing infection. Fixed equipment can not be easily sterilized between uses, and thus it is advantageous to provide sterile disposable handle covers which cover portions of the equipment which may be touched by personnel. In a particular non-limiting example, surgical lamps in an operating room environment are routinely covered with disposable handle covers, since during operating procedures medical personnel may desire to readjust their location manually.

The vast majority of such handles are cylindrical in nature, and thus are equally comfortable to adjust irrespective of the relative location of the equipment and the personnel. However, no standard exists for the size of the handles, and thus a range of diametric dimensions are found. Furthermore, the handles may be purely cylindrical, i.e. having a uniform diameter along the longitudinal axis thereof, or conically shaped, with a varying diameter along the longitudinal axis thereof.

A number of types of handle covers are commercially available, however their construction often leads to either disappointing results, or increased cost. For example, US 7,757,352 issued July 20, 2010 to Halamish et al., is addressed to sterile handle covers, utilizes a set of variable length spikes to secure a flexible bag to the target handle. Due to its non-uniformity, the requirement for variable length spikes adds to cost.

### SUMMARY OF THE INVENTION

In view of the discussion provided above and other considerations, the present disclosure provides methods and apparatus to overcome some or all of the disadvantages of prior and present handle covers. In one exemplary embodiment the embodiments enable an apparatus arranged to cover a target handle, the apparatus comprising: a flexible bag open at one end; an annular skirt; and a securing ring, the annular skirt comprising: an inner ring shaped edge, the inner ring shaped edge defining a longitudinal axis of the annular skirt; and a plurality of skirt fingers arrayed radially about the inner ring shaped edge and extending from the inner ring shaped edge towards the longitudinal axis of the annular skirt, the plurality of skirt fingers extending uniformly to a first predetermined radial distance from the longitudinal axis of the annular skirt, the extension of each of the plurality of skirt fingers attached to the extension of the adjacent skirt finger by a respective at least one skirt connection member, the at least one skirt connection member in cooperation with the plurality of skirt fingers defining at least one skirt band set about the longitudinal axis of the annular skirt at a radial distance at least equal to the first predetermined radial distance and less than the radius of the inner ring shaped edge, the securing ring arranged to mate securely with the annular skirt and thereby secure the open portion of the flexible bag, the securing ring comprising a plurality of securing ring fingers arrayed radially about, and extending from, an inner perimeter of a securing ring edge towards the longitudinal axis of the annular skirt when the securing ring is mated with the annular skirt, the plurality of securing ring fingers extending uniformly to a second predetermined radial distance from the longitudinal axis of the annular skirt, the extension of each of the plurality of securing ring fingers attached to the extension of the adjacent securing ring finger by a respective securing ring connection member, the securing ring connection members in cooperation with the plurality of securing ring fingers defining at least one securing ring band set about the longitudinal axis of the annular skirt at a radial distance at least equal to the second predetermined radial distance and less than the radius of the inner perimeter of the securing ring edge.

At least one of the skirt connection members and securing ring connection members is broken when the apparatus is set to cover the target handle. In another embodiment, the annular skirt extends convexingly from the inner rang shaped edge when viewed from the securing ring.

In one embodiment, the securing ring further comprises: a flange proceeding from a base portion of the securing ring to at least partially cover a face of the annular skirt. In another embodiment, the annular skirt further comprises a skirt collar extending from the annular skirt along the inner ring shaped edge parallel with the longitudinal axis, the securing ring arrange to mate in cooperation with the skirt collar.

In one embodiment, the second predetermined radial distance is greater than the first the predetermined radial distance. In another embodiment, the at least one skirt band comprises an inner and an outer skirt band, the inner skirt band at a radial distance from the longitudinal axis at least equal to the first predetermined radial distance and less than the radial distance of the skirt band, and the outer skirt band having a radial distance greater than the radial distance of the inner skirt band. In yet another embodiment, the second predetermined radial distance is greater than the first predetermined radial distance, and wherein the securing ring band is at a radial distance greater than the radial distance of the inner skirt band and less than the radial distance of the outer skirt band.

Independently, the present embodiments enable a method of providing a sterile cover for a handle, the method comprising: unfolding a folded sterile bag, with the open end of the unfolded sterile bag directed towards the handle, wherein the unfolding is responsive to an annular skirt assembly secured to the sterile bag being slid along the handle, the annular skirt assembly comprising: an annular skirt; and a securing ring, the annular skirt comprising: an inner ring shaped edge, the inner ring shaped edge defining a longitudinal axis of the annular skirt; and a plurality of skirt fingers arrayed radially about the inner ring shaped edge and extending from the inner ring shaped edge towards the longitudinal axis of the annular skirt, the plurality of skirt fingers extending uniformly to a first predetermined radial distance from the longitudinal axis of the annular skirt, the extension of each of the plurality of skirt fingers attached to the extension of the adjacent skirt finger by a respective at least one skirt connection member, the at least one skirt connection member in cooperation with the plurality of skirt fingers defining at least one skirt band set about the longitudinal axis of the annular skirt at a radial distance at least equal to the first predetermined radial distance and less than the radius of the inner ring shaped edge, the securing ring arranged to mate securely with the annular skirt and thereby secure the open portion of the sterile flexible bag, the securing ring comprising a plurality of securing ring fingers arrayed radially about, and extending from, an inner perimeter of a securing ring edge towards the longitudinal axis of the annular skirt when the securing ring is mated with the annular skirt, the plurality of securing ring fingers extending uniformly to a second predetermined radial distance from the longitudinal axis of the annular skirt, the extension of each of the plurality of securing ring fingers attached to the extension of the adjacent securing ring finger by a respective securing ring connection member, the securing ring connection members in cooperation with the plurality of securing ring fingers defining at least one securing ring band set about the longitudinal axis of the annular skirt at a radial distance at least equal to the second predetermined radial distance and less than the radius of the inner perimeter of the securing ring edge.

At least one of the skirt connection members and securing ring connection members is broken when the apparatus is slid along the handle. In yet another embodiment, the plurality of skirt fingers apply uniform pressure to the handle. In yet another embodiment, the plurality of securing ring fingers apply uniform pressure to the handle.

Additional features and advantages of the invention will become apparent from the following drawings and description.

### BRIEF DESCRIPTION OF DRAWINGS

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
FIGs. 1A - 1B illustrate various high level views of an annular skirt, according to certain embodiments;
FIGs. 2A - 2B illustrate various high level views of a securing ring, according to certain embodiments;
FIGs. 3A - 3B illustrate various high level views of a target handle cover apparatus, comprising a flexible bag, the annular skirt of FIGs. 1A - 1B and the securing ring of FIGs. 2A - 2B, according to certain embodiments;
FIG. 4A illustrates a high level view of a handle;
FIGs. 4B - 4C illustrate various high level views of the apparatus of FIGs. 3A - 3B covering the handle of FIG. 4A; and
FIG. 5 illustrates a high level flow chart of a method of providing a sterile cover for a handle.

### DESCRIPTION OF EMBODIMENTS

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting. The invention is defined by the appended independent claims. Preferred embodiment are given in the dependent claims.

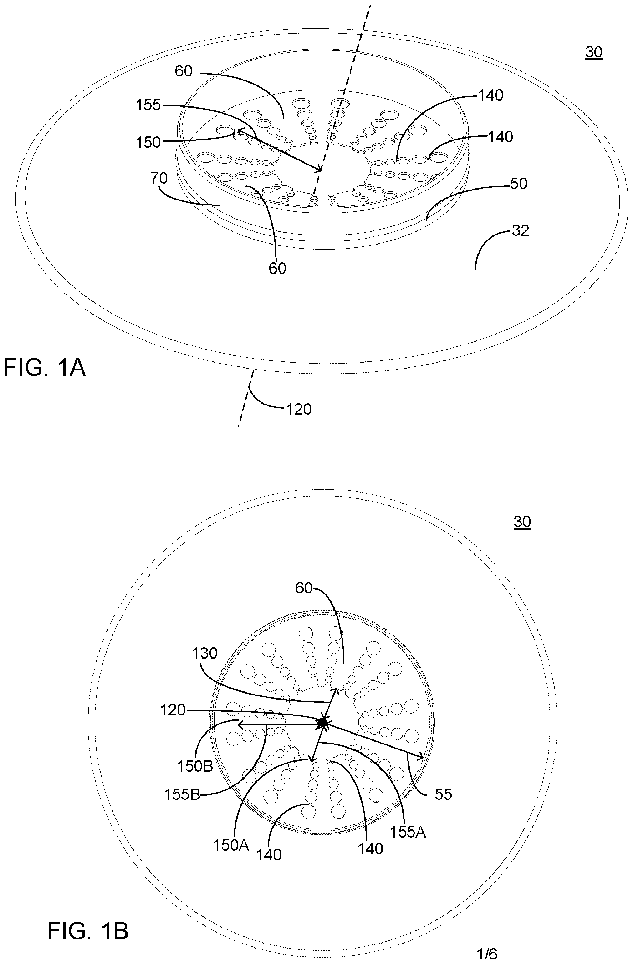

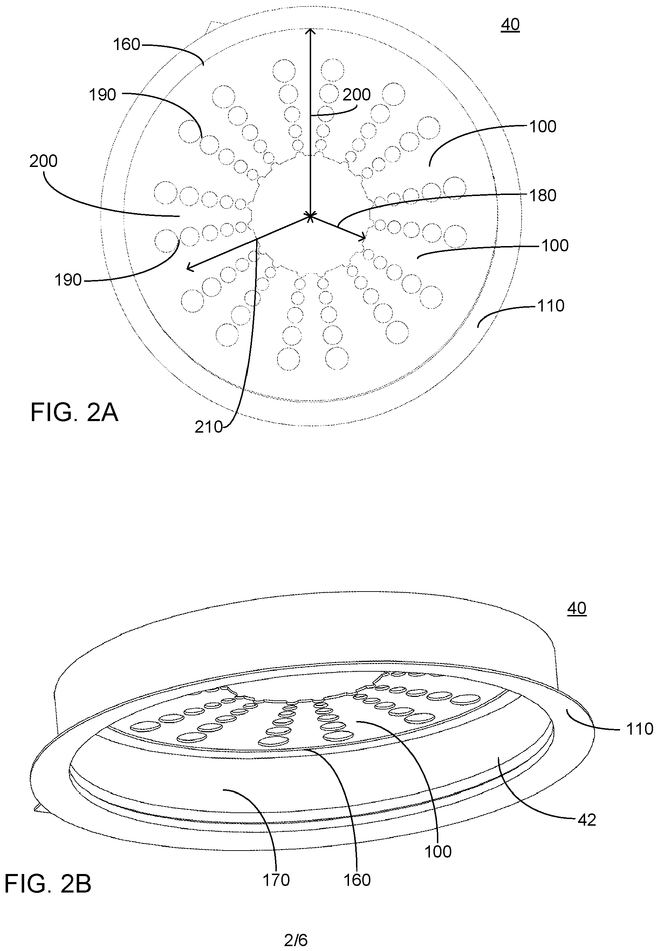

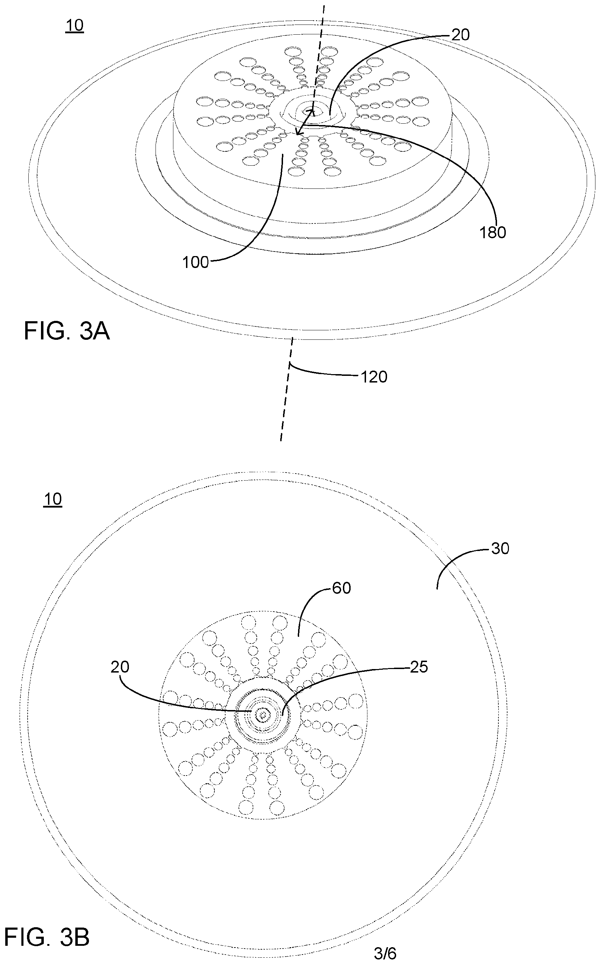

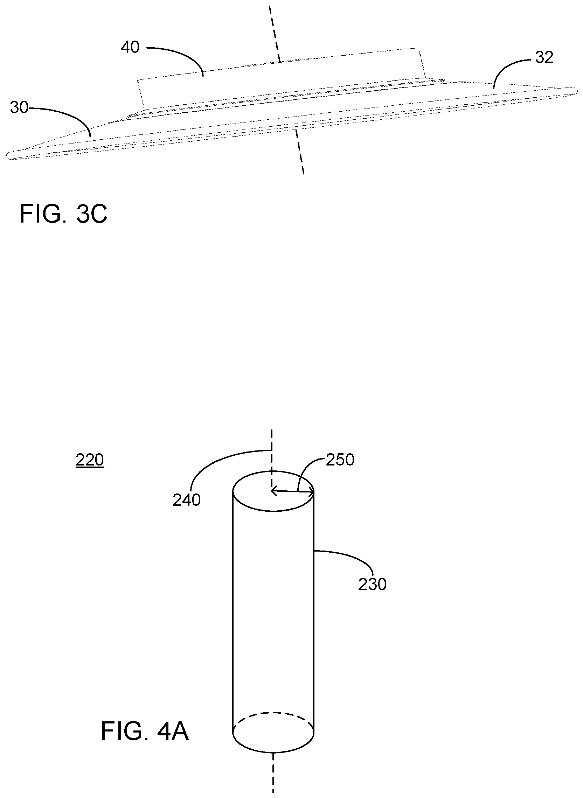

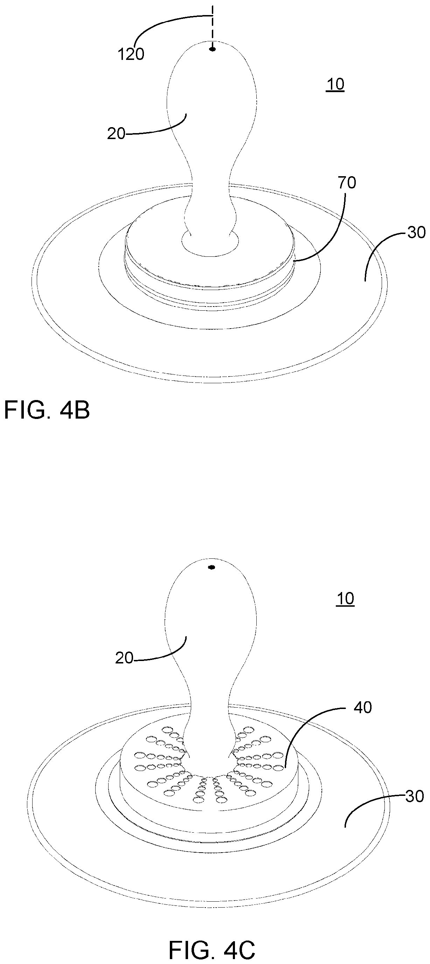

FIGs. 1A - 4C illustrate various high level views of a target handle cover apparatus 10, comprising: a flexible bag 20; an annular skirt 30; and a securing ring 40 exhibiting a radius 45. In particular, FIG. 1A illustrates a high level perspective view of annular skirt 30; FIG. 1B illustrates a high level top view of annular skirt 30; FIG. 2A illustrates a high level top view of securing ring 40; FIG. 2B illustrates a high level perspective view of securing ring 40; FIG. 3A illustrates a high level perspective view of apparatus 10; FIG. 3B illustrates a high level bottom view of apparatus 10; FIG. 4A illustrates a high level perspective view of a handle; FIG. 4B illustrates a high level perspective view of apparatus 10 covering handle 220, without securing ring 40; and FIG. 4C illustrates a high level perspective view of apparatus 10 covering handle 220, with securing ring 40, FIGs. 1A - 4C being described together. Annular skirt 30 comprises: an inner ring shaped edge 50 exhibiting a radius 55; a plurality of skirt fingers 60; and a skirt collar 70. Securing ring 40 comprises: a plurality of securing ring fingers 100; and a flange 110.

Inner ring shaped edge 50 defines a longitudinal axis 120 of annular skirt 30, longitudinal axis 120 extending through the center of inner ring shaped edge 50. Skirt fingers 60 are arranged radially about inner ring shaped edge 50 and extend from inner ring shaped edge 50 towards longitudinal axis 120. Skirt fingers 60 extend uniformly to a first predetermined radial distance 130 from longitudinal axis 120. The extension of each skirt finger 60 is attached to the extension of an adjacent skirt finger 60 by a respective at least one skirt connection member 140. In one embodiment, the extension of each skirt finger 60 is attached to the extension of an adjacent skirt finger 60 by a plurality of skirt connection members 140, optionally two skirt connection members 140.

At least one skirt connection member 140, in cooperation with skirt fingers 60, define at least one skirt band 150 set about longitudinal axis 120 of annular skirt 30 at a radial distance 155 at least equal to first radial distance 130 and less than radius 55 of inner ring shaped edge 50, i.e. at least one skirt band 150 exhibits a radius 155 greater than, or equal to, first radial distance 130 and less than radius 55. In one embodiment, at least one skirt band 150 comprises an inner skirt band 150A, set about longitudinal axis 120 at a radial distance 155A, and an outer skirt band 150B, set about longitudinal axis 120 at a radial distance 155B, greater than radial distance 155A. In another embodiment, more than two inner skirt bands 150 are provided, each exhibiting a respective radial distance 155 from longitudinal axis 120. Skirt collar 70 extends from a face 32 of annular skirt 30, along inner ring shaped edge 50, parallel with longitudinal axis 120. Face 32 of annular skirt 30 extends convexingly from inner ring shaped edge 50.

A base portion 42 of securing ring 40 is arranged to mate securely with annular skirt 30 over skirt collar 70. Securing ring fingers 100 are arranged radially about an edge 160 of securing ring 40 and extend from an inner perimeter 170 of edge 160 towards longitudinal axis 120 of annular skirt 30 when securing ring 40 is mated with annular skirt 30. Securing ring fingers 100 extend uniformly to a second predetermined radial distance 180 from longitudinal axis 120, the extension of each securing ring finger 100 attached to the extension of an adjacent securing ring finger 100 by a respective securing ring connection member 190. In one embodiment, second radial distance 180 of securing ring fingers 100 is greater than first radial distance 130 of skirt fingers 60. Securing ring connection members 190, in cooperation with securing ring fingers 100, define at least one securing ring band 200 set about longitudinal axis 120 at a radial distance 210 at least equal to second radial distance 180 and less than radius 45 of securing ring 40. Flange 110 proceeds from base portion 42 of securing ring 40 to at least partially cover face 32 of annular skirt 30.

Flexible bag 20 is open at an end 25 thereof, end 25 of flexible bag 20 positioned over second end 52 of inner ring shaped edge 50. As described above, securing ring 40 is arranged to mate securely with annular skirt 30 over second end 52 of inner ring shaped edge 50 to thereby secure open end 25 of flexible bag 20. In one embodiment (not shown) a plurality of catches are arrayed about skirt collar 70, the plurality of catches each arranged to mate with a respective space between adjacent securing ring fingers 100 and thereby secure open end 25 of flexible bag 20 and securing 40 to annular skirt 30. In one further embodiment (not shown), annular skirt 30 exhibits a plurality of cut-outs, each juxtaposed with a respective one of the plurality of catches, and arranged to provide spring-like action for the respective catches.

Apparatus 10 is arranged to provide a sterile cover for a handle 220. Annular skirt 30 and securing ring 40 are slid along the length 230 of handle 220 through open end 25 of flexible bag 20, a longitudinal axis 240 of handle 220 mating with longitudinal axis 120. At least one of skirt bands 150 and/or securing ring band 200 is broken when slid over handle 220. In one embodiment, first radial distance 130 of skirt fingers 60 and second radial distance 180 of securing ring fingers 100 are determined such that a radius 250 of handle 220 is greater than first radial distance 130 and second radial distance 180. Handle 220 applies pressure against skirt fingers 60 and securing ring fingers 100, thereby breaking at least one of skirt bands 150 and/or securing ring band 200. In the embodiment where an inner skirt band 150A and an outer skirt band 150B are provided, a handle 220 exhibiting a radius 250 less than a first predetermined length breaks inner skirt band 150A and a handle 220 exhibiting a radius 250 greater than the first predetermined length breaks both inner skirt band 150A and outer skirt band 150B.

The partial breaking of each band allows for the respective fingers to apply pressure to the handle. In particular, breaking some of the securing ring connection members 190 of securing ring band 200 causes securing ring fingers 100 to bend and apply pressure to handle 220 responsive to the remaining portion of securing ring band 200. Similarly, breaking some of the skirt connection members 140 of skirt band 150 causes skirt fingers 60 to bend and apply pressure to handle 220 responsive to the remaining portion of skirt band 150. Breaking inner skirt band 150A causes skirt fingers 60 to bend and apply pressure to handle 220, however if annular skirt 30 slides over a handle 220 exhibiting a radius 250 greater than the first predetermined length, handle 220 completely breaks inner skirt band 150A, i.e. all skirt connection members 140 thereof are broken, thereby skirt fingers 60 would not apply pressure to handle 220. Advantageously, only some of the skirt connection members 140 of outer skirt band 150B are broken, thereby skirt fingers 60 apply pressure to handle 220 responsive to the remaining portion of outer skirt band 150B. Therefore, skirt fingers 60 are arranged to apply uniform pressure to handles 220 exhibiting a wide range of radii 250. Particularly, pressure is applied to a handle 220 exhibiting a small radius 250 responsive to inner skirt band 150A and pressure is applied to a handle 220 exhibiting a large radius 250 responsive to outer skirt band 150B. Preferably, skirt fingers 60 are all composed of a single material therefore the pressure applied thereby to handle 220 is uniform. In one embodiment, during the manufacturing process, skirt fingers 60 are formed by perforating a single piece of material to thereby form skirt fingers 60 and the respective skirt bands 150.

As described above, securing ring fingers 100 are arranged to apply pressure to handle 220 when securing ring connection members 190 of securing ring band 200 is broken. Preferably, securing ring fingers 100 are all composed of a single material therefore the pressure applied thereby to handle 220 is uniform. In one embodiment, during the manufacturing process, securing ring fingers 100 are formed by perforating a single piece of material to thereby form securing ring fingers 100 and securing ring band 200.

Skirt fingers 60 and securing ring fingers 100 apply pressure to handle 220, thereby holding apparatus 10 in place on handle 220 and flexible bag 20 is arranged to cover handle 220. Preferably, apparatus 10 is sterile thereby providing a sterile cover for handle 220.

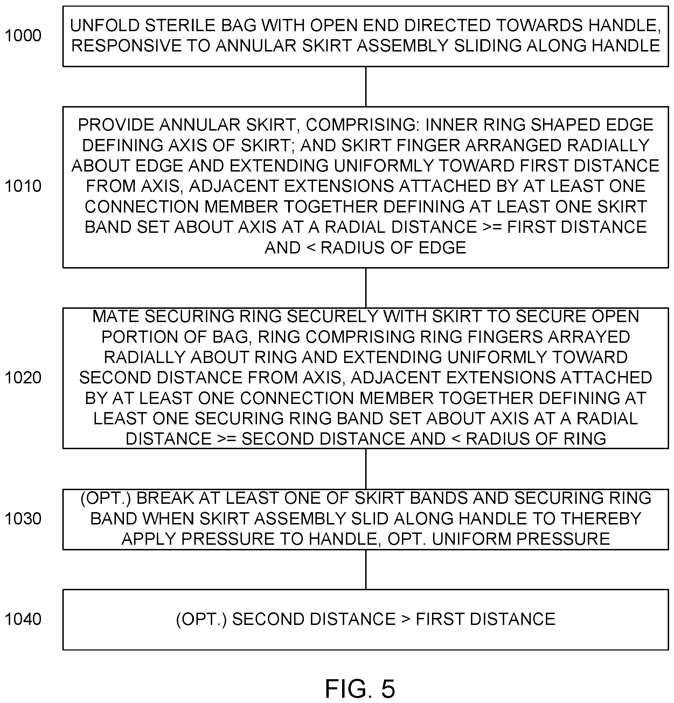

FIG. 5 illustrates a high level flow chart of a method of providing a sterile cover for a handle, according to certain embodiments. In stage 1000, a folded sterile bag is unfolded, with the open end of the sterile bag directed towards the handle. The unfolding is responsive to an annular skirt assembly, secured to the sterile bag, sliding along the handle. In stage 1010, an annular skirt is provided, the annular skirt comprising: an inner ring shaped edge defining a longitudinal axis of the annular skirt; and a plurality of skirt fingers arrayed radially about the inner ring shaped edge and extending from the inner ring shaped edge towards the longitudinal axis of the annular skirt. The plurality of skirt fingers extend uniformly to a first predetermined radial distance from the longitudinal axis of the annular skirt, the extension of each of the plurality of skirt fingers attached to the extension of the adjacent skirt finger by a respective at least one skirt connection member. The at least one skirt connection member, in cooperation with the plurality of skirt fingers, define at least one skirt band set about the longitudinal axis of the annular skirt at a radial distance at least equal to the first predetermined radial distance and less than the radius of the inner ring shaped edge.

In stage 1020, the securing ring of stage 1010 is arranged to mate securely with the annular skirt and thereby secure an open portion of the sterile flexible bag of stage 1000. The securing ring comprises a plurality of securing ring fingers arrayed radially about the securing ring and extending from an inner perimeter of the securing ring edge towards the longitudinal axis of the annular skirt when the securing ring is mated with the annular skirt. The plurality of securing ring fingers extend uniformly to a second predetermined radial distance from the longitudinal axis of the annular skirt, the extension of each of the plurality of securing ring fingers attached to the extension of the adjacent securing ring finger by a respective securing ring connection member, the securing ring connection members, in cooperation with the plurality of securing ring fingers, define at least one securing ring band set about the longitudinal axis of the annular skirt at a radial distance at least equal to the second predetermined radial distance and less than the radius of the inner perimeter of the securing ring edge.

In optional stage 1030, at least one of the skirt connection members of at least one of the skirt bands and/or at least one of the securing ring connection members of at least one of the securing ring bands breaks when the annular skirt assembly of stage 1000 is slid along the handle. The breaking of connection members of a skirt band and/or a securing ring band causes the respective skirt fingers and/or securing ring fingers to apply pressure to the handle and hold it in place. Optionally, as described above, the skirt fingers are all composed of the same material therefore the pressure applied thereby to the handle is uniform. Further optionally, as described above, the securing ring fingers are all composed of the same material therefore the pressure applied thereby to the handle is uniform. In optional stage 1040, the second predetermined radial distance of the securing ring fingers of stage 1020 is greater than the first predetermined radial distance of the skirt fingers of stage 1010.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

Unless otherwise defined, all technical and scientific terms used herein have the same meanings as are commonly understood by one of ordinary skill in the art to which this invention belongs. Although methods similar or equivalent to those described herein can be used in the practice or testing of the present invention, suitable methods are described herein.

It will be appreciated by persons skilled in the art that the present invention is not limited to what has been particularly shown and described hereinabove. Rather the scope of the present invention is defined by the appended claims and includes both combinations and sub-combinations of the various features described hereinabove as well as variations and modifications thereof, which would occur to persons skilled in the art upon reading the foregoing description.

## Claims

1. An apparatus (10) arranged to cover a target handle (220), the apparatus comprising:
a flexible bag (20) open at one end (25);
an annular skirt (30); and a
securing ring (40), said securing ring arranged to mate securely with said annular skirt and thereby secure the open end of said flexible bag,
wherein said annular skirt comprises:
an inner ring shaped edge (50), said inner ring shaped edge defining a longitudinal axis (120) of said annular skirt; and
a plurality of skirt fingers (60) arrayed radially about said inner ring shaped edge and extending from the inner ring shaped edge towards the longitudinal axis of said annular skirt,
wherein said securing ring comprises a plurality of securing ring fingers (100) arrayed radially about, and extending from, an inner perimeter (170) of a securing ring edge (160) towards the longitudinal axis of said annular skirt when said securing ring is mated with said annular skirt, **CHARACTERIZED BY**:
said plurality of skirt fingers (60) extending uniformly to a first predetermined radial distance (130) from the longitudinal axis of said annular skirt, the extension of each of said plurality of skirt fingers attached to the extension of the adjacent skirt finger by a respective at least one skirt connection member (140);
said at least one skirt connection member in cooperation with said plurality of skirt fingers defining at least one skirt band (150) set about the longitudinal axis of said annular skirt at a radial distance (155) at least equal to the first predetermined radial distance (130) and less than the radius (55) of said inner ring shaped edge (50);
said plurality of securing ring fingers (100) extending uniformly to a second predetermined radial distance (180) from the longitudinal axis (120) of said annular skirt, the extension of each of said plurality of securing ring fingers attached to the extension of the adjacent securing ring finger by a respective securing ring connection member (190); and
said securing ring connection members in cooperation with said plurality of securing ring fingers defining at least one securing ring band (200) set about the longitudinal axis of said annular skirt at a radial distance (210) at least equal to the second predetermined radial distance and less than the radius (45) of the inner perimeter of the securing ring edge,
wherein at least one of said at least one skirt band (150) and at least one securing ring band (200) is broken when the apparatus is set to cover the target handle (220) and the target handle applies pressure to at least one of said plurality of skirt fingers (60) and plurality of securing ring fingers (100).

2. The apparatus (10) according to claim 1, wherein said annular skirt (30) extends convexingly from said inner ring shaped edge (50) when viewed from said securing ring (40).

3. The apparatus (10) according claim 1, wherein the securing ring (40) further comprises:
a flange (110) proceeding from a base portion (42) of said securing ring to at least partially cover a face (32) of said annular skirt (30).

4. The apparatus (10) according to claim 1, wherein said annular skirt (30) further comprises a skirt collar (70) extending from said annular skirt along said inner ring shaped edge (50) parallel with said longitudinal axis (120), said securing ring (40) arrange to mate in cooperation with said skirt collar.

5. The apparatus (10) of any of claims 1-4, wherein the second predetermined radial distance (180) is greater than the first predetermined radial distance (130).

6. The apparatus (10) of any of claims 1 - 4, wherein said at least one skirt band (150) comprises an inner (150A) and an outer (150B) skirt band, the inner skirt band at a radial distance (155A) from said longitudinal axis at least equal to the first predetermined radial distance and less than the radial distance (155B) of said outer skirt band.

7. The apparatus (10) of claim 6, wherein the second predetermined radial distance (180) is greater than the first predetermined radial distance (130), and wherein the securing ring band (200) is at a radial distance (210) greater than the radial distance (155A) of said inner skirt band (150A) and less than the radial distance (155B) of said outer skirt band (150B).

8. A method of providing a sterile cover for a handle (220), the method comprising:
unfolding (1000) a folded sterile bag (20), with the open end (25) of the unfolded sterile bag directed towards the handle, wherein said unfolding is responsive to an annular skirt assembly (10) secured to the sterile bag being slid along the handle,
said annular skirt assembly comprising:
an annular skirt (30); and
a securing ring (40), said securing ring arranged to mate securely with said annular skirt and thereby secure the open portion of the sterile flexible bag,
wherein said annular skirt comprises:
an inner ring shaped edge (50), said inner ring shaped edge defining a longitudinal axis (120) of said annular skirt;
a plurality of skirt fingers (60) arrayed radially about said inner ring shaped edge and extending from the inner ring shaped edge towards the longitudinal axis of said annular skirt,
wherein said securing ring comprises a plurality of securing ring fingers (100) arrayed radially about, and extending from, an inner perimeter (170) of a securing ring edge (160) towards the longitudinal axis of said annular skirt when said securing ring is mated with said annular skirt, **CHARACTERIZED BY**:
said plurality of skirt fingers (60) extending uniformly to a first predetermined radial distance (130) from the longitudinal axis of said annular skirt, the extension of each of said plurality of skirt fingers attached to the extension of the adjacent skirt finger by a respective at least one skirt connection member (140);
said at least one skirt connection member in cooperation with said plurality of skirt fingers defining at least one skirt band (150) set about the longitudinal axis of said annular skirt at a radial distance (155) at least equal to the first predetermined radial distance (130) and less than the radius (55) of said inner ring shaped edge (50);
said plurality of securing ring fingers (100) extending uniformly to a second predetermined radial distance (180) from the longitudinal axis (120) of said annular skirt, the extension of each of said plurality of securing ring fingers attached to the extension of the adjacent securing ring finger by a respective securing ring connection member (190); and
said securing ring connection members in cooperation with said plurality of securing ring fingers defining at least one securing ring band (200) set about the longitudinal axis of said annular skirt at a radial distance (210) at least equal to the second predetermined radial distance and less than the radius (45) of the inner perimeter of the securing ring edge,
wherein at least one of said at least one skirt band (150) and at least one securing ring band (200) is broken when the apparatus is slid along the handle (220) and the handle applies pressure to at least one said plurality of skirt fingers (60) and plurality of securing ring fingers (100).

9. The method of claim 8, wherein said plurality of skirt fingers (60) apply uniform pressure (1030) to the handle (220).

10. The method of claim 8, wherein said plurality of securing ring fingers (100) apply uniform pressure (1030) to the handle (220).

## Patentansprüche

1. Vorrichtung (10), die dafür angeordnet ist, einen Zielgriff (220) abzudecken, wobei die Vorrichtung Folgendes umfasst:
einen flexiblen Beutel (20), der an einem Ende (25) offen ist,
einen kranzförmigen Rand (30) und
einen Sicherungsring (40), wobei der Sicherungsring dafür angeordnet ist, sicher mit dem kranzförmigen Rand zusammenzupassen und dadurch das offene Ende des flexiblen Beutels zu sichern,
wobei der kranzförmige Rand Folgendes umfasst:
eine innere ringförmige Kante (50), wobei die innere ringförmige Kante eine Längsachse (120) des kranzförmigen Randes definiert, und
mehrere Randfinger (60), die in Radialrichtung um die innere ringförmige Kante angeordnet sind und sich von der inneren ringförmigen Kante aus zu der Längsachse des kranzförmigen Randes hin erstrecken,
wobei der Sicherungsring mehrere Sicherungsringfinger (100) umfasst, die in Radialrichtung um einen Innenumfang (170) einer Sicherungsringkante (160) gruppiert sind und sich von demselben aus zu der Längsachse des kranzförmigen Randes hin erstrecken, wenn der Sicherungsring mit dem kranzförmigen Rand zusammengepasst ist, **dadurch gekennzeichnet, dass**:
sich die mehreren Randfinger (60) gleichförmig bis zu einem ersten vorbestimmten radialen Abstand (130) von der Längsachse des kranzförmigen Randes erstrecken, wobei die Verlängerung jedes der mehreren Randfinger durch ein jeweiliges, wenigstens ein Randverbindungselement (140) an der Verlängerung des benachbarten Randfingers befestigt ist,
das wenigstens eine Randverbindungselement im Zusammenwirken mit den mehreren Randfingern wenigstens ein Randband (150) definiert, das um die Längsachse des kranzförmigen Randes gelegt ist, in einem radialen Abstand (155), der dem ersten vorbestimmten radialen Abstand (130) wenigstens gleich und geringer als der Radius (55) der inneren ringförmigen Kante (50) ist,
sich die mehreren Sicherungsringfinger (100) gleichförmig bis zu einem zweiten vorbestimmten radialen Abstand (180) von der Längsachse (120) des kranzförmigen Randes erstrecken, wobei die Verlängerung jedes der mehreren Sicherungsringfinger durch ein jeweiliges Sicherungsring-Verbindungselemeht (190) an der Verlängerung des benachbarten Sicherungsringfingers befestigt ist, und
die Sicherungsring-Verbindungselemente im Zusammenwirken mit den mehreren Sicherungsringfingern wenigstens ein Sicherungsringband (200) definieren, das um die Längsachse des kranzförmigen Randes gelegt ist, in einem radialen Abstand (210), der dem zweiten vorbestimmten radialen Abstand wenigstens gleich und geringer als der Radius (45) des Innenumfangs der Sicherungsringkante ist,
wobei wenigstens eines von dem wenigstens einen Randband (150) und dem wenigstens einen Sicherungsringband (200) zerbrochen wird, wenn die Vorrichtung eingesetzt wird, um den Zielgriff (220) abzudecken, und der Zielgriff Druck auf wenigstens einen von den mehreren Randfingern (60) und den mehreren Sicherungsringfingern (100) ausübt.

2. Vorrichtung (10) nach Anspruch 1, wobei sich der kranzförmige Rand (30), von dem Sicherungsring (40) aus gesehen, konvex von der inneren ringförmigen Kante (50) aus erstreckt.

3. Vorrichtung (10) nach Anspruch 1, wobei der Sicherungsring (40) ferner Folgendes umfasst:
einen Flansch (110), der von einem Basisabschnitt (42) des Sicherungsrings vorspringt, um eine Fläche (32) des kranzförmigen Randes (30) wenigstens teilweise abzudecken.

4. Vorrichtung (10) nach Anspruch 1, wobei der kranzförmige Rand (30) ferner einen Randbund (70) umfasst, der sich von dem kranzförmigen Rand aus entlang der inneren ringförmigen Kante (50) parallel zu der Längsachse (120) erstreckt, wobei der Sicherungsring (40) dafür angeordnet ist, im Zusammenwirken mit dem Randbund zusammenzupassen.

5. Vorrichtung (10) nach einem der Ansprüche 1 bis 4, wobei der zweite vorbestimmte radiale Abstand (180) größer ist als der erste vorbestimmte radiale Abstand (130).

6. Vorrichtung (10) nach einem der Ansprüche 1 bis 4, wobei das wenigstens eine Randband (150) ein inneres (150A) und ein äußeres (150B= Randband umfasst, wobei sich das innere Randband in einem radialen Abstand (155A) von der Längsachse befindet, der wenigstens gleich dem ersten vorbestimmten radialen Abstand und geringer als der radiale Abstand (155B) des äußeren Randbandes ist.

7. Vorrichtung (10) nach Anspruch 6, wobei der zweite vorbestimmte radiale Abstand (180) größer ist als der erste vorbestimmte radiale Abstand (130) und wobei sich das Sicherungsringband (200) in einem radialen Abstand (210) befindet, der größer als der radiale Abstand (155A) des inneren Randbandes (150A) und geringer als der radiale Abstand (155B) des äußeren Randbandes (150B) ist.

8. Verfahren zum Bereitstellen einer sterilen Abdeckung für einen Griff (220), wobei das Verfahren Folgendes umfasst:
Entfalten (1000) eines gefalteten sterilen Beutels (20), wobei das offene Ende (25) des entfalteten sterilen Beutels zu dem Griff hin gerichtet ist, wobei das Entfalten als Reaktion darauf erfolgt, dass eine kranzförmige Randbaugruppe (10), die an dem sterilen Beutel befestigt ist, entlang des Griffs geschoben wird,
wobei die kranzförmige Randbaugruppe Folgendes umfasst:
einen kranzförmigen Rand (30) und
einen Sicherungsring (40), wobei der Sicherungsring dafür angeordnet ist, sicher mit dem kranzförmigen Rand zusammenzupassen und dadurch das offene Ende des flexiblen Beutels zu sichern,
wobei der kranzförmige Rand Folgendes umfasst:
eine innere ringförmige Kante (50), wobei die innere ringförmige Kante eine Längsachse (120) des kranzförmigen Randes definiert, und
mehrere Randfinger (60), die in Radialrichtung um die innere ringförmige Kante angeordnet sind und sich von der inneren ringförmigen Kante aus zu der Längsachse des kranzförmigen Randes hin erstrecken,
wobei der Sicherungsring mehrere Sicherungsringfinger (100) umfasst, die in Radialrichtung um einen Innenumfang (170) einer Sicherungsringkante (160) gruppiert sind und sich von demselben aus zu der Längsachse des kranzförmigen Randes hin erstrecken, wenn der Sicherungsring mit dem kranzförmigen Rand zusammengepasst ist, **dadurch gekennzeichnet, dass**:
sich die mehreren Randfinger (60) gleichförmig bis zu einem ersten vorbestimmten radialen Abstand (130) von der Längsachse des kranzförmigen Randes erstrecken, wobei die Verlängerung jedes der mehreren Randfinger durch ein jeweiliges, wenigstens ein Randverbindungselement (140) an der Verlängerung des benachbarten Randfingers befestigt ist,
das wenigstens eine Randverbindungselement im Zusammenwirken mit den mehreren Randfingern wenigstens ein Randband (150) definiert, das um die Längsachse des kranzförmigen Randes gelegt ist, in einem radialen Abstand (155), der dem ersten vorbestimmten radialen Abstand (130) wenigstens gleich und geringer als der Radius (55) der inneren ringförmigen Kante (50) ist,
sich die mehreren Sicherungsringfinger (100) gleichförmig bis zu einem zweiten vorbestimmten radialen Abstand (180) von der Längsachse (120) des kranzförmigen Randes erstrecken, wobei die Verlängerung jedes der mehreren Sicherungsringfinger durch ein jeweiliges Sicherungsring-Verbindungselement (190) an der Verlängerung des benachbarten Sicherungsringfingers befestigt ist, und
die Sicherungsring-Verbindungselemente im Zusammenwirken mit den mehreren Sicherungsringfingern wenigstens ein Sicherungsringband (200) definieren, das um die Längsachse des kranzförmigen Randes gelegt ist, in einem radialen Abstand (210), der dem zweiten vorbestimmten radialen Abstand wenigstens gleich und geringer als der Radius (45) des Innenumfangs der Sicherungsringkante ist,
wobei wenigstens eines von dem wenigstens einen Randband (150) und dem wenigstens einen Sicherungsringband (200) zerbrochen wird, wenn die Vorrichtung am Griff (220) entlang geschoben wird und der Zielgriff Druck auf wenigstens einen von den mehreren Randfingern (60) und den mehreren Sicherungsringfingern (100) ausübt.

9. Verfahren nach Anspruch 8, wobei die mehreren Randfinger (60) einen gleichförmigen Druck (1030) auf den Griff (220) ausüben.

10. Verfahren nach Anspruch 8, wobei die mehreren Sicherungsringfinger (100) einen gleichförmigen Druck (1030) auf den Griff (220) ausüben.

## Revendications

1. Appareil (10) agencé pour couvrir une poignée cible (220), l'appareil comprenant :
une poche souple (20) ouverte au niveau d'une extrémité (25) ;
une jupe annulaire (30) ; et une
bague de fixation (40), ladite bague de fixation étant agencée pour s'accoupler de manière fixe à ladite jupe annulaire et ainsi fixer l'extrémité ouverte de ladite poche souple,
ladite jupe annulaire comprenant :
un bord en forme de bague interne (50), ledit bord en forme de bague interne définissant un axe longitudinal (120) de ladite jupe annulaire ; et
une pluralité de doigts de jupe (60) agencés radialement autour dudit bord en forme de bague interne et s'étendant depuis le bord en forme de bague interne vers l'axe longitudinal de ladite jupe annulaire,
ladite bague de fixation comprenant une pluralité de doigts de bague de fixation (100) agencés radialement autour d'un et s'étendant depuis un périmètre interne (170) d'un bord de bague de fixation (160) vers l'axe longitudinal de ladite jupe annulaire lorsque ladite bague de fixation est accouplée à ladite jupe annulaire, **caractérisé par** :
ladite pluralité de doigts de jupe (60) s'étendant de manière uniforme sur une première distance radiale prédéterminée (130) depuis l'axe longitudinal de ladite jupe annulaire, l'extension de chacun de ladite pluralité de doigts de jupe étant fixée à l'extension du doigt de jupe adjacent par au moins un élément de liaison de jupe respectif (140) ;
ledit au moins un élément de liaison de jupe conjointement avec ladite pluralité de doigts de jupe définissant au moins un ensemble de bande de jupe (150) autour de l'axe longitudinal de ladite jupe annulaire à une distance radiale (155) au moins égale à la première distance radiale prédéterminée (130) et inférieure au rayon (55) dudit bord en forme de bague interne (50) ;
ladite pluralité de doigts de bague de fixation (100) s'étendant de manière uniforme sur une seconde distance radiale prédéterminée (180) depuis l'axe longitudinal (120) de ladite jupe annulaire, l'extension de chacun de ladite pluralité de doigts de bague de fixation étant fixée à l'extension dudit doigt de bague de fixation adjacent par un élément de liaison de bague de fixation respectif (190) ; et
lesdits éléments de liaison de bague de fixation conjointement à ladite pluralité de doigts de bague de fixation définissant au moins un ensemble de bande de bague de fixation (200) autour de l'axe longitudinal de ladite jupe annulaire à une distance radiale (210) au moins égale à la seconde distance radiale prédéterminée et inférieure au rayon (45) du périmètre interne du bord de bague de fixation,
au moins l'une de ladite au moins une bande de jupe (150) et de l'au moins une bande de bague de fixation (200) étant rompue lorsque l'appareil est configuré pour couvrir la poignée cible (220) et la poignée cible applique une pression sur au moins l'un de ladite pluralité de doigts de jupe (60) et de ladite pluralité de doigts de bague de fixation (100).

2. Appareil (10) selon la revendication 1, ladite jupe annulaire (30) s'étendant de manière convexe depuis ledit bord en forme de bague interne (50) lorsqu'il est vu depuis ladite bague de fixation (40).

3. Appareil (10) selon la revendication 1, ladite bague de fixation (40) comprenant en outre :
une bride (110) provenant d'une partie de base (42) de ladite bague de fixation pour couvrir au moins partiellement un côté (32) de ladite jupe annulaire (30).

4. Appareil (10) selon la revendication 1, ladite jupe annulaire (30) comprenant en outre un collier de jupe (70) s'étendant depuis ladite jupe annulaire le long dudit bord en forme de bague interne (50) parallèle audit axe longitudinal (120), ladite bague de fixation (40) étant agencée pour s'accoupler conjointement audit collier de jupe.

5. Appareil (10) selon l'une quelconque des revendications 1 à 4, la seconde distance radiale prédéterminée (180) étant supérieure à la première distance radiale prédéterminée (130).

6. Appareil (10) selon l'une quelconque des revendications 1 à 4, ladite au moins une bande de jupe (150) comprenant une bande de jupe interne (150A) et externe (150B), la bande de jupe interne étant à une distance radiale (155A) depuis ledit axe longitudinal au moins égale à la première distance radiale prédéterminée et inférieure à la distance radiale (155B) de ladite bande de jupe externe.

7. Appareil (10) selon la revendication 6, la seconde distance radiale prédéterminée (180) étant supérieure à la première distance radiale prédéterminée (130), et la bande de bague de fixation (200) étant à une distance radiale (210) supérieure à la distance radiale (155A) de ladite bande de jupe interne (150A) et inférieure à la distance radiale (155B) de ladite bande de jupe externe (150B).

8. Procédé de fourniture d'une couverture stérile pour une poignée (220), le procédé comprenant :
le dépliage (1000) d'une poche stérile pliée (20), avec l'extrémité ouverte (25) de la poche stérile dépliée dirigée vers la poignée, ledit dépliage répondant à un ensemble jupe annulaire (10) fixé à la poche stérile coulissant le long de la poignée,
ledit ensemble jupe annulaire comprenant :
une jupe annulaire (30) ; et
une bague de fixation (40), ladite bague de fixation étant agencée pour s'accoupler de manière fixe à ladite jupe annulaire et ainsi fixer la partie ouverte de la poche souple stérile,
ladite jupe annulaire comprenant :
un bord en forme de bague interne (50), ledit bord en forme de bague interne définissant un axe longitudinal (120) de ladite jupe annulaire ;
une pluralité de doigts de jupe (60) agencés radialement autour dudit bord en forme de bague interne et s'étendant depuis le bord en forme de bague interne vers l'axe longitudinal de ladite jupe annulaire ;
ladite bague de fixation comprenant une pluralité de doigts de bague de fixation (100) agencés radialement autour d'un et s'étendant depuis un périmètre interne (170) d'un bord de bague de fixation (160) vers l'axe longitudinal de ladite jupe annulaire lorsque ladite bague de fixation est accouplée à ladite jupe annulaire, **caractérisé par** :
ladite pluralité de doigts de jupe (60) s'étendant de manière uniforme sur une première distance radiale prédéterminée (130) depuis l'axe longitudinal de ladite jupe annulaire, l'extension de chacun de ladite pluralité de doigts de jupe étant fixée à l'extension du doigt de jupe adjacent par au moins un élément de liaison de jupe respectif (140) ;
ledit au moins un élément de liaison de jupe conjointement avec ladite pluralité de doigts de jupe définissant au moins un ensemble de bande de jupe (150) autour de l'axe longitudinal de ladite jupe annulaire à une distance radiale (155) au moins égale à la première distance radiale prédéterminée (130) et inférieure au rayon (55) dudit bord en forme de bague interne (50) ;
ladite pluralité de doigts de bague de fixation (100) s'étendant de manière uniforme sur une seconde distance radiale prédéterminée (180) depuis l'axe longitudinal (120) de ladite jupe annulaire, l'extension de chacun de ladite pluralité de doigts de bague de fixation étant fixée à l'extension du doigt de bague de fixation adjacent par un élément de liaison de bague de fixation respectif (190) ; et
lesdits éléments de liaison de bague de fixation conjointement avec ladite pluralité de doigts de bague de fixation définissant au moins un ensemble de bande de bague de fixation (200) autour de l'axe longitudinal de ladite jupe annulaire à une distance radiale (210) au moins égale à la seconde distance radiale prédéterminée et inférieure au rayon (45) du périmètre interne du bord de bague de fixation,
au moins l'une de ladite au moins une bande de jupe (150) et de l'au moins une bande de bague de fixation (200) étant rompue lorsque l'appareil coulisse le long de la poignée (220) et la poignée applique une pression sur au moins l'un de ladite pluralité de doigts de jupe (60) et de ladite pluralité de doigts de bague de fixation (100).

9. Procédé selon la revendication 8, ladite pluralité de doigts de jupe (60) appliquant une pression uniforme (1030) sur la poignée (220).

10. Procédé selon la revendication 8, ladite pluralité de doigts de bague de fixation (100) appliquant une pression uniforme (1030) sur la poignée (220).
